(19) Europäisches Patentamt
European Patent Office
Office européen des brevets

(11) Veröffentlichungsnummer: **0 000 772**
**A1**

# (12) EUROPÄISCHE PATENTANMELDUNG

(21) Anmeldenummer: 78100581.4

(22) Anmeldetag: 02.08.78

(51) Int. Cl.²: G 01 N 33/16, C 08 F 8/00

(30) Priorität: 03.08.77 CH 9542/77

(43) Veröffentlichungstag der Anmeldung: 21.02.79 Patentblatt 79/4

(84) Benannte Vertragsstaaten: BE CH DE FR GB NL SE

(71) Anmelder: F.Hoffmann-La Roche & Co. Aktiengesellschaft
Abt. VIII-Pt
CH-4002 Basel(CH)

(72) Erfinder: Gaetano, Roncari, Dr.
Im Leberngarten 9
CH-4107 Ettingen(CH)

(74) Vertreter: Lederer, Franz, Dr. et al,
Patentanwälte Lederer, Meyer Lucile-Grahn-Strasse 22
D-8000 München 80(DE)

## (54) Immunologisches Reagenz in Form spezieller, mit einem immunologisch aktiven Material beschichteter Latexteilchen auf Vinylpolymerisatbasis, Verfahren zu dessen Herstellung, Verwendung dieses Reagenzes, Bestimmungsverfahren unter Verwendung dieses Reagenzes und Reagenziengarnitur enthaltend dieses Reagenz.

(57) Immunologisches Reagenz, Verfahren zu dessen Herstellung, Verwendung dieses Reagenzes, Bestimmungsverfahren unter Verwendung dieses Reagenzes und Reagenziengarnitur enthaltend dieses Reagenz.

Es wurde ein immunologisches Reagenz mit einem aus Partikeln von Vinylpolymerisaten gebildeten Träger hergestellt, mit dem keine unerwünschte Vernetzung des eingesetzten Antikörpers oder Antigens eintritt. Dieser Träger bildet mit einem breiten Spektrum von immunologiech aktiven Materialien ein diagnostisch verwendbares Reagenz, das Stabil, spezifisch und empfindlich ist. Der Träger liegt in Form diskreter Latexteilchen vor; der Latex besteht aus einer Dispersion von Partikeln von Vinylpolymerisaten, die als Endgruppen Gruppen der Formel:

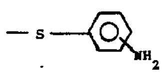

tragen, wobei die Partikel aus einem Kern von Vinyl- und/oder Dienpolymerisat, das Carboxyl- und/oder Sulfonatfunktionen trägt, und aus einer äusseren Schicht von Vinylpolymerisat, das als Endgruppen Gruppen der Formel:

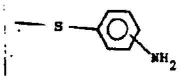

trägt, gebildet sind und einen mittleren Durchmesser zwischen 0,03 und 5 µm haben, zu den zu verwendenden immunologisch aktiven Substanzen gehören Amine, Aminosäuren, Peptide, Proteine, Lipoproteine, Glycoproteide, Sterine, Steroide, Lipoide, Nucleinsäuren, Enzyme, Hormone, Vitamine, Polysaccharide und Alkaloide.

EP 0 000 772 A1

0000772

2. Aug. 1978

RAN 4093/ 32

F. Hoffmann-La Roche & Co. Aktiengesellschaft, Basel/Schweiz

Immunologisches Reagenz in Form spezieller, mit einem immunologisch aktiven Material beschichteter Latexteilchen auf Vinylpolymerisatbasis, Verfahren zu dessen Herstellung, Verwendung dieses Reagenzes, Bestimmungsverfahren unter Verwendung dieses Reagenzes und Reagenziengarnitur enthaltend dieses Reagenz.

Die vorliegende Erfindung betrifft wertvolle diagnostische Reagenzien, ein Verfahren zu deren Herstellung und diagnostische Methoden, worin solche Reagenzien Verwendung finden.

Die Diagnose von pathologischen oder anderen Zuständen in Menschen und Tieren wird oft unter Anwendung von immunologischen Prinzipien durchgeführt. Diese Prinzipien werden zum Nachweis von Antikörpern oder Antigenen in den Körperflüssigkeiten des Lebewesens benützt. Ein Antigen ist eine fremde Substanz, welche, wenn sie dem Lebewesen appliziert wird, die Bildung von gewissen löslichen und als Antikörper bezeichneten Substanzen bewirkt. Irgendeine Substanz wie z.B. ein Protein, welche normal nicht in einem bestimmten Lebewesen vorhanden ist, kann die Bildung von Antikörpern verursachen, wenn sie dem Lebewesen unter geeigneten Bedingungen appliziert wird.

Hen

Nach ihrer Bildung reagieren die Antikörper mit den Antigenen und schützen auf diese Weise, im Fall eines Bakterien- oder Virus-Fremdkörpers, gegen Infektionen.

Immunologische Testverfahren beruhen auf der Antigen-Antikörper-Reaktion, welche sich gewöhnlich durch Unlöslichkeit oder Agglutination manifestiert.

Im allgemeinen wird die Anwesenheit eines Antigens oder eines Antikörpers dadurch bestätigt oder bestimmt, dass man den entsprechenden Antikörper oder das entsprechende Antigen einer Körperflüssigkeit des Lebewesens, meistens Urin, Blutserum oder einem speziell behandelten Blutextrakt, zugibt. Es können jedoch auch andere Körperflüssigkeiten verwendet werden. Man stellt die Anwesenheit bzw. die Abwesenheit des Antikörpers oder des Antigens in der Körperflüssigkeit des Lebewesens fest, indem das Eintreten oder nicht-Eintreten einer Antigen-Antikörperreaktion festgestellt wird.

Weil einige Komplexe sich nur sehr langsam bilden und sehr geringe Teilchengrössen besitzen, ist es notwendig, Träger zu benützen, um sie sichtbar zu machen. In einer bisher bevorzugten Methode wurden der Antikörper bzw. das Antigen mittels eines Carbodiimids über eine Amidbindung an diskreten Teilchen von carboxylierten Latexpolymeren, wie z.B. von carboxylierten Copolymeren aus Butadien und Styrol, gebunden.

Diese Methode hat jedoch den Nachteil, dass während der Kupplung des Proteins (Antikörper oder Antigen) an die Latexteilchen, wegen der Verwendung von Carbodiimiden als Nebenreaktion eine unerwünschte Vernetzung des eingesetzten Proteins eintritt und somit ein Teil der oft sehr teuren Proteine für die Kupplung mit dem Träger verloren geht.

Die vorliegende Erfindung betrifft nun neuartige polymere Träger, mit welchen die obigen Nachteile vermieden werden können und welche mit einem breiten Spektrum von immunologisch aktiven

Materialien ein diagnostisch verwendbares Reagenz bilden können, das stabil, spezifisch und empfindlich ist und eine leicht nachweisbare visuelle Bewertung in sehr kurzer Zeit ermöglicht.

Genauer gesagt betrifft die vorliegende Erfindung ein wasserunlösliches Reagenz für eine immunologische Bestimmung mit einem etwa dem von Wasser entsprechenden spezifischen Gewicht in Form diskreter Latexteilchen, an welche ein immunologisch aktives Material gebunden ist, dadurch gekennzeichnet, dass der Latex aus einer Dispersion von Partikeln von Vinylpolymerisaten besteht, die als Endgruppen Gruppen der Formel:

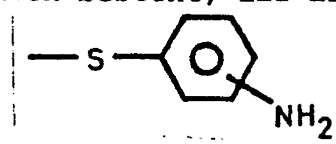

tragen, wobei die Partikel aus einem Kern von Vinyl- und/oder Dienpolymerisat, das Carboxyl- und/oder Sulfonatfunktionen trägt, und aus einer äusseren Schicht von Vinylpolymerisat, das als Endgruppen Gruppen der Formel:

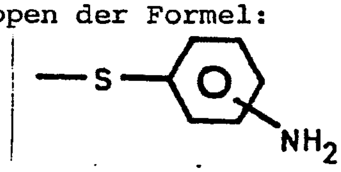

trägt, gebildet sind und einen mittleren Durchmesser zwischen 0,03 und 5 µm haben.

Weiter bezieht sich die Erfindung auf ein Verfahren zur Herstellung eines solchen Reagens, welches dadurch gekennzeichnet ist, dass man den Latex nach Diazotierung oder in Gegenwart von geeigneten bifunktionellen Reagenzien mit dem immunologisch aktiven Material umsetzt.

Als "immunologisch aktive Substanzen" können all jene Bestandteile in physiologischen Flüssigkeiten, Zell- und Gewebeextrakten genannt werden, für die ein immunologischer Reaktionspartner vorhanden ist oder gebildet werden kann. Dazu gehören Amine, Aminosäuren, Peptide, Proteine, Lipoproteine, Glycoproteide, Sterine, Steroide, Lipoide, Nucleinsäuren, Enzyme, Hormone, Vitamine, Polysaccharide und Alkaloide. Bevorzugte

immunologisch aktive Substanzen sind in der folgenden Tabelle zusammengestellt:

Tabelle I

I. Antigene gewonnen aus Mikroorganismen

Bakterien

1. Gram-positive Kokken
   Streptokokken (Pyogene, Fecalis und Viridans)
   Staphylokokken (aureus und albus)
   Pneumokokken (D. pneumoniae)

2. Gram-negative Kokken
   Neisseria (gonorrhoeae und meningitidis)

3. Gram-positive, aerobe Bazillen
   Bacillus anthracis
   Corynebacterium diphtheriae
   Erysipelothrix
   Listeria monocytogenes

4. Gram-positive, anaerobe Bazillen
   Clostridia (botulinum, perfringens, welchii und tetani)

5. Gram-negative, anaerobe Bazillen
   Bacteroides

6. Gram-negative, intestinale Bazillen
   Escherichia
   Klebsiella
   Enterobacter
   Proteus
   Pseudomonas
   Salmonella
   Shigella

7. Gram-negative, nicht intestinale Bazillen
   Pasteurella (pestis und tularensis)
   Hemophilus influenzae
   Brucella (melitensis, abortus und suis)
   Bordetella pertussis
   Malleomyces

8. Spirocheten
   Treponema pallidum
   Leptospira
   Borrelia

9. Mycoplasma

10. Mycobakterien

11. Vibrio

12. Actinomyces

Protozoen

1. Intestinale Protozoen
   Amöben

2. Flagellates
   Trichomonas
   Leishmania
   Trypanosomes
   Toxoplasma (T. Gondii)

3. Sporozoen
   Plasmodia (vivax, falciparum, malariae und ovale)

Pilze

1. Sporotrichum

2. Cryptococcus

3. Blastomyces

4. Histoplasma

5. Coccidioides

6. Candida

Viren und Rickettsien

1. Rickettsia

2. Viren
   Hundehepatidis
   Shope papilloma
   Influenza A & B
   Hühnerpest
   Herpes simplex
   Adenoviren
   Polyoma
   Rous sarcoma
   Vaccinia
   Poliovirus
   Röteln
   Hundestaupe
   Leukemia
   Mumps
   Newcastle-Krankheit (Haushuhnkrankheit)
   Sendai
   ECHO
   Maul- und Klauenseuche

Psittacosis
Rabis
Ectromelia
Arborviren

II. Fremde Antigene

Polysaccharide
Hyaluronidase
Tetanus-toxin
Eiovalbumin
Schafserumalbumin
menschliches Plasma-gamma-globulin
menschliches Serum-albumin

III. Natürliche Antigene

1. Hormone

Insulin
Glucagon
Thyroid-Hormone
Choriongonadotropin
Chorion-Wachstumshormon - Prolactin

2. Enzyme

Pancreas-chymotrypsinogen
Procarboxypeptidase
Deoxyribonuclease
Ribonuclease
Catalase
Creatin-Phosphokinase

3. Organspezifische Antigene

Niere
Leber
Haut
Herz (Myoglobin)
gastrointestinaler Trakt
Prostata
Embryo-Antigene (z.B. CEA-Antigen)
Tumor-Antigene

4. Bindegewebekomponenten

Muskel
Collagen
Amyloid

5. Blutzellenantigene, Blutgruppensubstanzen und andere Isoantigene

Plättchen
Megakaryocyten
Leucocyten
Erythrocyten
Blutgruppensubstanzen
Forssman-Antigen
Histoverträglichkeits-Antigene

6. Plasmaproteine

Fibrin und Fibrinoid
Plasminogen und Plasmin

7. Pathologische Globuline

Myeloma, makroglobulinaemische und dysglobulinaemische Proteine
Rheumatoidfaktor
C-reaktionsfähiges Protein

IV. Natürliche Antikörper

1. Natürliches Gammaglobulin

Natürliche Antikörper - nephrotoxische Antikörper
Komplement

2. Auto-Antikörper

antinuclearer Faktor
Thyroidautoantikörper
Adrenalautoantikörper
Autoantikörper für gastrisch-parietale Zellen bei perniziöser Anämie
Autoantikörper für Spermatozoen
Muskel-Autoantikörper bei Myasthenia gravis
Autoantikörper für Nervengewebe
Autoantikörper gegen faserartiges Gewebe und Vaskularkomponenten
Autoantikörper gegen Plättchen und Megakaryocyten
Antikörper gegen Trophoblasten

3. Induzierte Antikörper gegen:

Immunoglobulinklassen IgG, IgA, IgM oder unterschiedliche Spezies

V. Haptenische Verbindungen

Opiumalkaloid (Morphin)
Antipyrin
Barbitursäure

Im Rahmen der vorliegenden Erfindung sind besonders bevorzugte immunologisch aktive Substanzen Albumin, Rheumatoidfaktor, menschliches Immunoglobulin IgG und Antikörper gegen IgG.

Unter Vinylpolymerisaten, die den Kern der Partikeln bilden, versteht man Homopolymerisate von Monomeren, wie Styrol und seine Derivate: Methylstyrole, Aethylstyrole, Vinyltoluol; Vinylchlorid, Vinylidenchlorid; Vinylacetat; Acrylderivate, wie Alkylacrylate und -methacrylate (Alkyl mit 1 bis 10 Kohlenstoffatomen), die gegebenenfalls hydroxyliert sind, wie 2-Hydroxyäthylacrylat und -methacrylat und 2-Hydroxypropylacrylat und -methacrylat; Acrylnitril und Methacrylnitril; sowie Copolymerisate dieser Monomeren untereinander und/oder mit modifizierenden Vinylcomonomeren, wie Divinylbenzol, Acrylamid und Methacrylamid und deren N-substituierte Derivate, wie z.B. Methylolacrylamid; diese Comonomere stellen bis zu 5 Gew.-% des Copolymerisates dar.

Unter Dienpolymerisaten, die den Kern bilden, versteht man Homopolymerisate des Butadiens und seiner Derivate: Chloropren, Isopren; sowie die Copolymerisate dieser Monomeren untereinander und/oder mit Vinylmonomeren, wie sie oben genannt wurden, in allen Mengenverhältnissen und/oder mit modifizierend wirkenden Vinylmonomeren, wie sie oben aufgezählt wurden, deren Menge in dem Copolymerisat bis zu 5 Gew.-% ausmacht.

Die Vinylpolymerisate, welche die äussere Schicht der Partikel bilden, sind Homopolymerisate von Monomeren, wie Styrol und seine Derivate, z.B. Methylstyrole, Aethylstyrole

und Vinyltoluol; gegebenenfalls hydroxylierte Alkylacrylate und Alkylmethacrylate (Alkyl mit 1 bis 10 Kohlenstoffatomen); Acrylnitril und Methacrylnitril; sowie Copolymerisate dieser Monomeren untereinander und/oder mit modifizierend wirkenden Vinylcomonomeren, wie Divinylbenzol, Acrylamid und Methacrylamid sowie deren N-substituierte Derivate, wie Methylolacrylamid, die bis zu 5 Gew.-% des Copolymerisats ausmachen können.

In den Partikeln stellt das Kernpolymerisat 30 bis 99,5 Gew.-%, vorzugsweise 60 bis 99 Gew.-%, und das Polymerisat der äusseren Schicht 70 bis 0,5 Gew.-%, vorzugsweise 40 bis 1 Gew.-%, dar.

Die Polymerisatpartikel, deren Kornverteilung je nach den gewünschten Eigenschaften des Latex und den in Betracht gezogenen Anwendungen breit oder eng sein kann, haben einen mittleren Durchmesser zwischen 0,03 und 5 µm, vorzugsweise zwischen 0,05 und 1 µm. Sie stellen bis zu 60 Gew.-%, vorzugsweise bis zu 45 Gew.-%, des Latex dar. Jedoch kann der Latex ohne weiteres verdünnt oder konzentriert werden.

Das Kernpolymerisat kann hergestellt werden durch Emulsionspolymerisation des oder der Vinylmonomeren und/oder Dienmonomeren in Gegenwart von mindestens einer äthylenischen Mono- oder Polycarbonsäure, die mit dem oder den Monomeren copolymerisierbar ist, und/oder mindestens eines copolymerisierbaren ungesättigten Alkaliorganosulfonats; dann wird das Polymerisat der äusseren Schicht hergestellt durch Emulsionspolymerisation des oder der Vinylmonomeren in Gegenwart des Latex des Kernpolymerisates, der oben erhalten wurde, und in Gegenwart eines Kettenübertragungsmittels.

Die bei der Polymerisation des Kernpolymerisates und bei der Polymerisation des Polymerisates der äusseren Schicht verwendeten Monomeren sind die oben aufgezählten Monomeren. Sie werden entweder alle vor der Polymerisation verwendet oder zum

Teil vor der Polymerisation verwendet, wobei der restliche Teil im Verlauf der Polymerisation in aufeinanderfolgenden Fraktionen oder kontinuierlich zum Reaktionsmedium gegeben wird, oder alle im Verlauf der Polymerisation in aufeinanderfolgenden Fraktionen oder kontinuierlich zugesetzt.

Als copolymerisierbare äthylenische Mono- oder Polycarbonsäuren seien Acrylsäure, Methacrylsäure, Maleinsäure, Fumarsäure, Crotonsäure, Sorbinsäure, Zimtsäure, Itaconsäure, Aconitsäure genannt, die in Mengen zwischen 0,5 und 15 Gew.-%, vorzugsweise zwischen 0,5 und 10 Gew.-%, bezogen auf das oder die Monomeren, verwendet werden.

Die copolymerisierbaren ungesättigten Alkaliorganosulfonate sind z.B. Natriumvinylsulfonat, Natriummethallylsulfonat, Natrium-2-sulfoäthylacrylat, Natrium-2-sulfoäthylmetacrylat, 2-Acrylamido-2-methylpropansulfonat; sie werden in Mengen zwischen 0,1 und 3 Gew.-%, bezogen auf das oder die Monomeren, verwendet.

Die copolymerisierbaren äthylenischen Mono- oder Polycarbonsäuren und die copolymerisierbaren ungesättigten Alkaliorganosulfonate können einzeln oder in Kombination in den angegebenen Mengen verwendet werden.

Die Herstellung des Kernpolymerisates erfolgt in Emulsion nach jedem beliebigen klassischen Verfahren in Gegenwart eines Initiators und eines Emulgators.

Als Initiator verwendet man vorzugsweise Alkalipersulfate, wasserlösliche Diazoderivate oder Redoxysysteme auf Basis von Wasserstoffperoxyd, organischen Peroxyden oder Hydroperoxyden in Mengen in der Grössenordnung von 0,01 bis 5 Gew.-%, vorzugsweise 0,03 bis 3 Gew.-%, bezogen auf das oder die Monomeren.

Der verwendete Emulgator kann anionaktiv und/oder nichtionogen sein. Es handelt sich um klassische Produkte für die Emulsionspolymerisation.

Als anionaktive Emulgatoren seien genannt Salze von Fettsäuren; Alkalialkylsulfate, Alkalialkylsulfonate, Alkalialkylarylsulfonate, Alkalialkylsulfosuccinate, Alkalialkylphosphate; Sulfobernsteinsäurealkylester; Sulfonate von Alkylphenolpolyglycoläthern; Salze von Estern von Alkylsulfopolycarbonsäuren; Kondensationsprodukte von Fettsäuren mit Oxyalkansulfonsäuren und Aminoalkansulfonsäuren; sulfatierte Derivate von Polyglycoläthern; sulfatierte Ester von Fettsäuren und Polyglycolen; Alkanolamide von sulfatierten Fettsäuren.

Als nichtionogene Emulgatoren kommen Fettsäureester von Polyalkoholen, Alkanolamide von Fettsäuren, Polyäthylenoxyde, Copolyäthylenoxyd/Propylenoxyd, oxyäthylierte Alkylphenole in Betracht.

Die zu verwendenden Mengen des oder der Emulgatoren liegen in der Grössenordnung von 0,01 bis 5 Gew.-%, bezogen auf das oder die Monomeren, und ihre Einführung erfolgt entweder insgesamt vor der Polymerisation oder zum Teil vor der Polymerisation, wobei der restliche Teil im Verlauf der Polymerisation in nacheinanderfolgenden Fraktionen oder kontinuierlich zum Reaktionsmedium zugesetzt wird, oder insgesamt im Verlauf der Polymerisation in nacheinanderfolgenden Fraktionen oder kontinuierlich.

Die für die Polymerisation des Kernpolymerisates zu verwendende Menge Wasser muss derart sein, dass die Konzentration des oder der Monomeren 60 Gew.-% nicht übersteigt.

Obgleich es nicht unbedingt erforderlich ist, ist es möglich, dem Reaktionsmedium beliebige Verbindungen zuzusetzen, die entweder die Ionenstärke des Mediums und demzufolge die Kornverteilung zu modifizieren vermögen, wie Mineralsalze, Elektrolyten, in einer Menge bis zu 3 Gew.-%, bezogen auf die Monomeren, oder die den pH-Wert des Mediums zu modifizieren vermögen, wie beispielsweise Puffer, Säuren, Basen. Jedoch

wurde in bestimmten Fällen festgestellt, dass es zur Begünstigung der Copolymerisation zu bevorzugen ist, wenn das Medium neutral oder sauer ist.

Die Polymerisationstemperatur, die eine Funktion des verwendeten Initiators und des herzustellenden Polymerisats ist, liegt im allgemeinen zwischen -5 und +90°C.

Die erhaltenen Latices weisen Polymerisatpartikel mit einem Durchmesser zwischen 0,03 und 5 µm, vorzugsweise zwischen 0,05 und 1 µm, auf. Diese Partikel sind im allgemeinen nicht kalibriert, aber es ist möglich, sie kalibriert zu erhalten, wenn man bekannte Kalibrierverfahren für die Emulsionspolymerisation anwendet, wie die gesteuerte Zugabe des Emulgators und/oder des oder der Monomeren und insbesondere die Animpfung. Im letzten Falle kann der Emulgator in dem Impfmaterial enthalten sein.

Die Partikel sind aus Homopolymerisat oder Copolymerisat mit einer Oberfläche von Carboxyl- und/oder Sulfonatfunktionen gebildet. Das Vorhandensein dieser Funktionen kann durch konduktometrische Titration bestätigt werden.

Die Herstellung des Polymerisates der äusseren Schicht wird in wässriger Emulsion in Gegenwart von Kernpolymerisat, Kettenübertragungsmittel, Initiator und gegebenenfalls Emulgator ausgeführt.

Die verwendete Menge des Kernpolymerisats liegt zwischen 30 und 99,5 Gew.-% und beträgt vorzugsweise 60 bis 99 Gew.-%, bezogen auf die Summe von Kernpolymerisat und zu polymerisierendem Monomer oder zu polymerisierenden Monomeren.

Das Kettenübertragungsmittel vom Typ Aminophenyldisulfid oder Aminophenylmercaptan ist insbesondere o,o'-Dithiobisanilin, p,p'-Dithiobisanilin, 2-Mercaptoanilin, 3-Mercaptoanilin, 4-Mercaptoanilin. Dieses Mittel wird im allgemeinen in Lösung

in dem oder den Monomeren verwendet, und zwar in Mengen zwischen 0,1 und 10 Gew.-%, vorzugsweise zwischen 0,5 und 5 Gew.-%, bezogen auf das oder die Monomere.

Die für die Polymerisation des oder der Monomeren der äusseren Schicht erforderlichen Initiatoren sind Diazoinitiatoren, Azonitrile wie Azo-bis-isobutyronitril oder wie sulfonierte Azonitrile, wie sie im französischen Patent Nr. 1.233.582 beschrieben sind; von diesen kann man erwähnen Azobis-(isobutyronitrilnatriumsulfonat), Azobis-(α-methylbutyronitrilnatriumsulfonat), Azobis-(α-methyl-β-äthoxycarbonylbutyronitrilnatriumsulfonat); carboxylierte Azonitrile, wie 4,4'-Azobis-(4-cyanpentansäure) und ihre Salze, Azobis-alkylamidiniumsalze, wie α,α'-Azobis-isobutyramidiniumchlorid, Azobis-N,N'-dimethylenisobutyramidiniumchlorid.

Der Initiator, der in einer Menge von 0,01 bis 3 Gew.-%, vorzugsweise 0,1 bis 2 Gew.-%, bezogen auf das oder die Monomeren, verwendet wird, wird insgesamt oder zum Teil vor der Polymerisation verwendet, wobei der andere Teil im Verlauf der Polymerisation in nacheinanderfolgenden Fraktionen oder kontinuierlich zu dem Reaktionsmedium zugesetzt wird, insbesondere wenn die Lebensdauer des Initiators bei der Polymerisationstemperatur kurz ist. Der Initiator kann auch insgesamt im Verlauf der Polymerisation kontinuierlich zum Reaktionsmedium zugegeben werden.

Der allfällige Emulgator wird aus anionaktiven und/oder nichtionogenen Emulgatoren gewählt, die für die Herstellung des Kernpolymerisats angegeben wurden; er kann gleich oder verschieden wie der für die Herstellung des Kernpolymerisates verwendete Emulgator sein. Er wird in Mengen von bis zu 10 Gew.-%, bezogen auf das oder die Monomeren, verwendet, und seine Einführung kann je nach dem mittleren Durchmesser der Latexpartikel, der erhalten werden soll, entweder insgesamt vor der Polymerisation oder zum Teil vor der Polymerisation erfolgen, wobei der restliche Teil im Verlauf der Polymerisation in nacheinanderfolgenden Fraktionen oder kontinuierlich zugegeben wird, oder sie kann insgesamt im Verlauf der Polymerisation in aufeinanderfolgenden Fraktionen oder kontinuierlich erfolgen.

Die bei der Polymerisation der äusseren Schicht zu verwendende Menge Wasser muss derart sein, dass die Konzentration von Kernpolymerisat und zu polymerisierendem oder zu polymerisierenden Monomeren 60 Gew.-%, vorzugsweise 45 Gew.-%, nicht übersteigt.

Die Polymerisationstemperatur, die eine Funktion des gewählten Initiators ist, liegt im allgemeinen zwischen 5 und 100°C, vorzugsweise zwischen 40 und 90°C.

Die erhaltenen Latices weisen Polymerisationspartikel auf, deren Durchmesser zwischen 0,03 und 5 µm, vorzugsweise zwischen 0,05 und 1 µm, liegt; da die Menge der äusseren Schicht nicht sehr gross ist, modifiziert sie nicht in merklicher Weise die Grösse der Partikel des Kernpolymerisates. Die Partikel können kalibriert sein oder nicht, aber bei bestimmten Anwendungen wird es aus Gründen der Reproduzierbarkeit bevorzugt, dass sie kalibriert sind, d.h. dass sie eine schmale Korngrössenverteilung haben.

Die Latices sind mechanisch und bei der Lagerung sowie gegen Elektrolyten beständig, d.h. sie flocken nicht aus, wenn man ihnen Mineralsalze, wie z.B. die Chloride, Nitrate, Borate, Phosphate des Natriums, Calciums, Magnesiums, Kaliums zusetzt.

Die Partikel sind aus Polymerisaten gebildet und weisen eine Oberfläche mit Carboxyl- und/oder Sulfonatfunktionen sowie Gruppen der Formel:

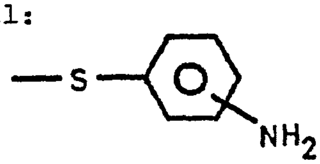

auf.

Obgleich die äussere Schicht auf dem Kernpolymerisat polymerisiert wird, bleiben die Carboxyl- und/oder Sulfonatfunktionen zugänglich, wie durch konduktometrische Titration gezeigt werden kann, und die Gruppen der Formel:

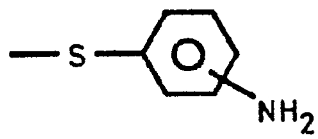

stehen für weitere Reaktionen zur Verfügung.

Die immunologisch aktiven Materialien (Antigen oder Antikörper) können physikalisch und/oder chemisch an den erfindungsgemäss verwendeten Latexpolymeren gebunden werden.

In einer Ausführungsform wird das erfindungsgemässe Reagenz durch Bildung einer Azobindung zwischen dem Latex und dem immunologisch aktiven Material hergestellt. Zu diesem Zweck werden durch Behandlung des Latex in wässriger saurer Lösung mit einem Nitrit die primären aromatischen Aminogruppen des Latex in ein Diazoniumsalz überführt.

Als Säure kann z.B. eine anorganische Säure wie Salzsäure, Schwefelsäure oder Perchlorsäure verwendet werden. Als Nitrit wird vorzugsweise Natriumnitrit oder Kaliumnitrit verwendet. Die Reaktion wird wegen der Instabilität der Diazoniumsalze vorzugsweise bei 0-5$^{o}$C ausgeführt.

Das immunologische aktive Material wird anschliessend im wässrigen Medium, vorzugsweise zwischen 0$^{o}$C und Zimmertemperatur,mit dem diazotierten Träger zur Reaktion gebracht.

In einer weiteren Ausführungsform des erfindungsgemässen Verfahrens kann das immunologisch aktive Material mit Hilfe einer polyfunktionellen Verbindung über ein Zwischenstück an den gemäss vorliegender Erfindung verwendeten Latex gebunden werden.

Als polyfunktionelle Verbindungen eignen sich diejenigen, welche mit den aromatischen Aminogruppen des Latexpolymeren reagieren oder an dem aromatischen Ring des Latexpolymeren eine Substitutionsreaktion eingehen und gleichzeitig mit funktionellen Gruppen des immunologisch aktiven Materials, wie Amino-, Mercapto-, Carboxyl- und Hydroxylgruppen, reagieren oder eine Substitutionsreaktion an dem aromatischen Ring des immunologisch aktiven Materials eingehen.

Repräsentative Vertreter derartiger polyfunktioneller Verbindungen sind Azo-, Isocyano-, Isothiocyano- oder Aldehydgruppen enthaltenden Verbindungen wie z.B. Bis-diazobenzidin, Bis-diazobenzidin-disulfonsäure, Bis-diazo-p-phenyldiamin, Phenyldiisocyanat, Toluoldiisocyanat, Glutardialdehyd.

Bei Umsetzung in Gegenwart einer bifunktionellen Verbindung wird das immunologisch aktive Material in wässrigem Medium, vorzugsweise bei Zimmertemperatur (20°C bis 25°C), mit dem Träger zur Reaktion gebracht. Die Temperatur kann jedoch auch zwischen 0°C und 40°C liegen.

Die Menge der verwendeten bifunktionellen Verbindung hängt ab von der Zahl der Aminogruppen auf dem Latex. Vorzugsweise wird ein zehn- bis hundert-facher molarer Ueberschuss der bifunktionellen Verbindung gegenüber der Zahl der Aminogruppen des eingesetztes Latex verwendet.

In beiden Ausführungsformen zur Herstellung des erfindungsgemässen Reagenz ist der pH-Wert der Reaktion wichtig. Er darf nicht so gewählt werden, dass ein Protein-Reaktionspartner denaturiert wird. In der Regel liegt der pH-Wert zwischen 5 und 9. Dieser pH-Wert wird unter Verwendung von geeigneten üblichen Puffer-Systemen wie Phosphatpuffer und dgl., aufrechterhalten.

Das Endprodukt ist ein wasserunlösliches Material, welches in einer wässrigen Pufferlösung vom pH-Wert 5,0 bis 9 suspendiert ist, wobei der pH-Wert der Lösung von dem im einzelnen

verwendeten System und von den Anforderungen an die Stabilität des immunologisch aktiven Materials abhängig ist. Das spezifische Gewicht des Produktes entspricht etwa demjenigen von Wasser (0,97-1,02), wodurch eine stabile Suspension des Produkts erreicht wird. Die Produkte können z.B. durch Zentrifugieren in Form eines weissen oder gelblichen Niederschlags isoliert werden.

Die Menge an immunologisch aktivem Material, das an die immunologisch inerten Latexpolymer-Träger gebunden ist, beträgt in der Regel 0,01 bis 15,0 Gew.%. Jedoch wird jedes einzelne immunologisch aktive Material in einer Menge benützt, welche sich in einem diagnostischen Test am zweckmässigsten erweist. Aus diesem Grunde wird jedes Material mit dem Träger in einem Verhältnis kombiniert, welches den jeweiligen spezifischen Anforderungen am besten entspricht. Die vorliegende Erfindung umfasst deshalb die Verwendung einer solchen Menge an immunologisch aktivem Material in Kombination mit einem immunologisch inerten Latexpolymer-Träger, die geeignet ist, ein für derartige diagnostische Zwecke nützliches Reagens zu liefern.

Nach seiner Herstellung kann das Produkt in spezifischen diagnostischen Tests, welche auf immunologischen Prinzipien aufgebaut sind, verwendet werden.

Erfindungsgemäss kann die Bestimmung der immunologisch aktiven Substanz sowohl in einem direkten wie auch in einem indirekten (Inhibitions-) Testverfahren durchgeführt werden.

Im direkten Testverfahren werden zur Bestimmung einer immunologisch aktiven Substanz die Analysenprobe und die mit dem entsprechenden immunologischen Reaktionspartner beschichteten Latexteilchen vermischt und das Auftreten einer Agglutination beobachtet. Der Test ist positiv, wenn eine Agglutination festgestellt wird.

Beim indirekten (Inhibitions-)-Testverfahren wird zur Bestimmung einer immunologisch aktiven Substanz die Analysenprobe mit einer bestimmten Menge des entsprechenden immunologischen Reaktionspartners(z.B. Antiserum) und Latexteilchen, die mit der immunologisch aktiven Substanz beschichtet sind, vermischt und das Auftreten einer Agglutination beobachtet. Der Test ist positiv, wenn keine Agglutination festgestellt wird.

Die in derartigen immunologischen Testverfahren nutzbaren Reagenzien können vorteilhaft für kommerzielle Zwecke in eine diagnostische Testgarnitur abgepackt werden.

Im Falle eines direkten Tests enthält die Reagenziengarnitur zur Bestimmung einer immunologisch aktiven Substanz in einem Behälter eine wässrige Suspension von mit dem entsprechenden immunologischen Reaktionspartner beschichteten Latexteilchen.

Im Falle eines indirekten Tests enthält die Reagenziengarnitur zur Bestimmung einer immunologisch aktiven Substanz in einem ersten Behälter eine Lösung des entsprechenden immunologischen Reaktionspartners (z.B. Antiserum) und in einem zweiten Behälter eine wässrige Suspension von mit dem immunologisch aktiven Material beschichteten Latexteilchen.

In beiden Fällen kann die wässrige Suspension des an Latex gebundenen immunologisch aktiven Materials oder des an Latex gebundenen immunologischen Reaktionspartners in irgendeiner Konzentration vorhanden sein. Jedoch ist eine Konzentration von 0,5 bis 5 Gew.% bevorzugt.

Die Erfindung wird anhand der folgenden Beispielen veranschaulicht.

## Beispiel 1

In einem Autoklaven von 25 Liter stellt man einen Latex von Kernpolymerisat her, wobei man verwendet:

4800 g entionisiertes Wasser
50 g Kaliumpersulfat
50 g Natriumpyrophosphat
10 g Natriumlaurylsulfat
50 g Natriummethallylsulfonat
100 g Acrylsäure
100 g Itaconsäure
2135 g Styrol
2865 g Butadien

Die Polymerisation wird bei 75°C unter Stickstoffatmosphäre ausgeführt, wobei die Monomeren im Verlauf von 7 Stunden kontinuierlich eingeführt werden und die Reaktion 8 Stunden lang fortgesetzt wird.

Nach dem Abkühlen erhält man einen Latex vom pH = 2,5, dessen Konzentration an Polymerisatpartikeln 51 Gew.-% beträgt.

Durch Elektronenmikroskopie stellt man fest, dass die Partikel einen mittleren Durchmesser von 0,145 µm haben; 90% der Partikel haben einen Durchmesser zwischen 0,14 und 0,15 µm.

Die Zusammensetzung des Polymerisates ist im wesentlichen gleich wie diejenige der verwendeten Monomeren. Die Partikel tragen auf ihrer Oberfläche Carboxyl- und Sulfonatfunktionen, die durch konduktometrische Titration bestimmt werden.

406 g des erhaltenen Latex und 1541 g entionisiertes Wasser werden in einen Reaktor eingeführt. Das Gemisch wird unter Rühren auf 70°C erhitzt; diese Temperatur wird während der ganzen Dauer der Reaktion aufrechterhalten.

Sobald das Gemisch 70°C erreicht hat, wird es unter Stickstoffatmosphäre gehalten; man gibt in 3 Stunden mit konstanter Geschwindigkeit gleichzeitig 1,25 g Natriumdihexylsulfosuccinat in 150 g Wasser, 0,20 g α,α'-Azobis-isobutyramidiniumchlorid in 210 g Wasser, 18 g Styrol, die 0,45 g p,p'-Dithiobisanilin enthalten, zu.

Dann wird die Polymerisation 5 Stunden lang fortgesetzt. Das Gemisch wird danach abgekühlt.

Eigenschaften des erhaltenen Latex:

pH: 3,1
Konzentration an Polymerisationspartikeln: 9,3 Gew.-%
Elektrolytbeständigkeit: 5
mittlerer Durchmesser der Partikel: 0,15 µm, wobei 90% einen Durchmesser zwischen 0,145 und 0,155 µm haben.

Die Partikel tragen auf ihrer Oberfläche Carboxyl- und Sulfonatfunktionen, die durch konduktometrische Titration bestätigt werden, und Gruppen der Formel:

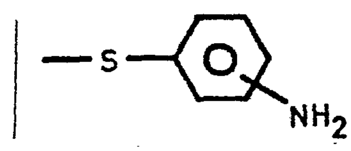

1 ml von dem oben hergestellten 10%igen Latex wird 20 ml Wasser zugesetzt und die Mischung 1 1/2 Stunden bei 35'000 g zentrifugiert. Der Ueberstand wird abdekantiert, der Rückstand in 20 ml Wasser aufgenommen und nochmals 1 1/2 Stunden bei 35'000 g zentrifugiert. Diese Operation wird zweimal wiederholt und der so erhaltene Latex wird in den folgenden Beispielen als "gewaschener Latex" bezeichnet.

5 mg humanes Immunoglobulin G (Cohn Fraktion II) wird durch Erhitzen auf 60°C während 3 Stunden in 1 ml 0,1 M Glycin-HCl Puffer pH 4 denaturiert.

1 ml gewaschener Latex wird 5 ml 0,05 M HCl zugesetzt und die Mischung auf 0°C abgekühlt. Zu dieser Mischung wird 0,1 ml einer 0,01 M $NaNO_2$ Lösung gegeben und es wird 15 Minuten bei 0°C gerührt. Der diazotierte Latex wird bei 5°C 1 1/2 Stunden bei 35'000 g zentrifugiert und der Ueberstand abdekantiert. Der Rückstand wird in 5 ml eisgekühltem 0,1 M Glycin-HCl Puffer pH 4,0 aufgenommen und im gleichen Puffer 1 ml 0,5%iges denaturiertes humanes Immunoglobulin G zugegeben, 1 Stunde im Eisbad gerührt und anschliessend über Nacht bei 10° stehen gelassen. Der Latex wird 1 1/2 Stunden bei 35'000 g abzentrifugiert, der Ueberstand abdekantiert und der Rückstand zweimal mit je 25 ml 0,1 M Glycin-NaOH Puffer pH 8,2 gewaschen, durch Zentrifugieren und Aufschlämmen des Rückstandes. Dem Latex wird nun soviel Puffer zugesetzt, dass eine Lösung mit 30 mg/ml resultiert.

Agglutinationstest:

Für den Röhrchen-Agglutinationstest wird der folgende Puffer verwendet: 7,5 g Glycin, 6,0 g $CaCl_2$, 3 g Rinderalbumin, 1 g $NaN_3$ gelöst in 1 Liter Wasser. Der pH-Wert wird mit NaOH auf 8,2 eingestellt. Für den Nachweis der Rheumafaktoren im Serum wird in einem kleinen Reagenzglas 20 µl Latex mit 3 ml Puffer verdünnt und 25 µl des zu untersuchenden Serums zugegeben. Nach dem Durchmischen werden die Röhrchen während 2 Stunden in einem Wärmeblock bei 37°C gehalten. Ein positives Serum agglutiniert unter diesen Bedingungen während ein negatives Kontrollsystem keine Agglutination zeigt.

Beispiel 2

1 ml gewaschener Latex wird wie in Beispiel 1 hergestellt und diazotiert. Dem diazotierten Latexrückstand wird 5

ml eiskalter 0,1 M Glycin-NaOH Puffer pH 6,0 zugesetzt und 5 mg Ziegen anti-Humanalbumin Immunoglobulin G in 1 ml obigem Puffer gelöst zugegeben und die Mischung wird nach Rühren während 1 Stunde bei 10°C über Nacht stehen gelassen. Der Latex wird bei 35'000 g während 1 1/2 Stunden abzentrifugiert, der Ueberstand verworfen und das Sediment zweimal mit je 25 ml 0,1 M Glycin-NaOH pH 8,2 gewaschen. Nach dem Waschen wird der Latex mit soviel Puffer vermischt, dass eine 3%ige Lösung erhalten wird.

Agglutinationstest:

Für den Röhrchenagglutinationstest wird der folgende Puffer verwendet: 7,5 g Glycin, 6,0 g $CaCl_2$, 3 g Rinderalbumin und 1,0 g $NaN_3$ werden in 1 Liter Wasser gelöst und das pH mit Salzsäure auf 6,0 eingestellt. Es wird in kleinen Reagenzgläsern eine Konzentrationsreihe von Humanalbumin in 3 ml Puffer erstellt, je 20 µl Latex zugegeben und nach Durchmischen 2 Stunden bei 37°C in einem Wärmeblock gehalten.

| 20 | 10 | 1 | 0,1 | 0,05 | 0,01 | 0 | µg Humanalbumin/ml Puffer |
|---|---|---|---|---|---|---|---|
| + | + | + | + | + | - | - | |

+ = agglutiniert in 2 Stunden
- = nicht agglutiniert in 2 Stunden

Aus dieser Tabelle ist ersichtlich, dass mit dem so hergestellten Latex 0,05 µg Humanalbumin/ml bestimmt werden kann.

Beispiel 3

1 ml gewaschenem Latex von Beispiel 1 wird 5 ml 0,1 M Phosphatpuffer pH 5,0 zugesetzt und 5 mg Schaf anti-Human IgG Immunoglobulin G in 1 ml Puffer zugegeben und es wird gut gerührt Anschliessend wird 0,1 ml einer 0,01 M p-Phenyldiisothiocyanatlösung in Dimethylformamid zugegeben, 1 Stunde gerührt und bei

Raumtemperatur über Nacht stehen gelassen. Der Latex wird 1 1/2 Stunden bei 35'000 g zentrifugiert, der Ueberstand verworfen und der Rückstand zweimal mit je 25 ml 0,1 M Glycin-NaOH Puffer pH 8,2 gewaschen. Für den Agglutinationstest wird der Latex in einer Konzentration von 30 mg/ml eingesetzt.

Agglutinationstest:

Für den Röhrchenagglutinationstest wird ein 0,1 M Phosphatpuffer pH 6,0 mit 0,1% Rinderalbumin verwendet. Es wird eine Konzentrationsreihe von Human IgG in 3 ml Puffer hergestellt. Zu jedem Röhrchen werden 20 µl Latexreagens gegeben, durchmischt und 2 Stunden bei 37°C inkubiert im Wärmeblock.

| 100 | 10 | 1 | 0,1 | 0,05 | 0 | µg Human IgG/ml Puffer |
|---|---|---|---|---|---|---|
| + | + | + | + | - | - | |

+ = agglutiniert in 2 Stunden
- = agglutiniert nicht in 2 Stunden

Die Tabelle zeigt, dass mit diesem Latexreagens 0,1 µg/ml Human IgG noch bestimmt werden können.

## Beispiel 4

36,8 mg Benzidin werden in 0,5 ml 2 NaCl gelöst und mit 7,5 ml Wasser verdünnt. Die Mischung wird im Eisbad gekühlt und unter Rühren 27,2 mg $NaNO_2$ in 2 ml Wasser zugetropft. Das so erhaltene bisdiazotierte Benzidin ist bei -20° während Wochen haltbar.

0000772

1 ml gewaschener Latex von Beispiel 1 wird in 5 ml 0,1 M Phosphatpuffer pH 7,0 aufgenommen und 5 mg humanes Immunoglobulin G in 1 ml obigem Puffer zugegeben. Die Suspension wird auf 0° abgekühlt und unter Rühren 0,01 ml einer 0,02 M bisdiazotierten Benzidinlösung zugegeben und anschliessend bei 10° über Nacht stehen gelassen. Der Latex wird bei 30'000 g während 1 1/2 Stunden zentrifugiert, der Ueberstand verworfen und das Sediment zweimal mit je 25 ml 0,1 M Glycin-NaOH pH 8,2 gewaschen. Nach dem Waschen wird der Latex wird soviel Puffer vermischt, dass eine 3%ige Lösung erhalten wird.

Agglutinationstest:

Für die Bestimmung von IgG im Inhibitionstest wird ein 0,1 M Phosphatpuffer pH 6,0 verwendet. In kleine Reagenzgläser wird je 3 ml eines 1/500 verdünnten Schaf anti-human IgG Serums und steigende Mengen von human IgG gegeben. Nach Inkubation während 15 Minuten bei 37°C wird in jedes Röhrchen 20 µl Latexreagenz gegeben und 3 Stunden bei 37°C inkubiert.

| 0,01 | 0,05 | 0,10 | 0,50 | 1,0 | 5,0 | µg human IgG/3ml |
|---|---|---|---|---|---|---|
| + | + | + | + | - | - | |

+ = agglutiniert in 3 Stunden
- = nicht agglutiniert in 3 Stunden

Es lassen sich mit dem oben beschriebenen Latexreagenz 0,16 µg human IgG/ml bestimmen.

0000772

RAN 4093/32

## Patentansprüche

1. Wasserunlösliches Reagenz für eine immunologische Bestimmung,mit einem etwa dem von Wasser entsprechenden spezifischen Gewicht in Form diskreter Latexteilchen, an welche ein immunologisch aktives Material gebunden ist, dadurch gekennzeichnet, dass der Latex aus einer Dispersion von Partikeln von Vinylpolymerisaten besteht, die als Endgruppen Gruppen der Formel:

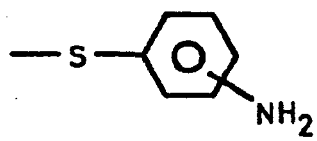

tragen, wobei die Partikel aus einem Kern von Vinyl- und/oder Dienpolymerisat, das Carboxyl- und/oder Sulfonatfunktionen trägt, und aus einer äusseren Schicht von Vinylpolymerisat, das als Endgruppen Gruppen der Formel:

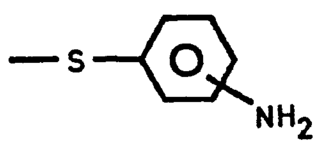

trägt, gebildet sind und einen mittleren Durchmesser zwischen 0,03 und 5 µm haben.

2. Reagenz nach Anspruch 1, dadurch gekennzeichnet, dass die Vinylpolymerisate entweder Homopolymerisate von Monomeren sind, die aus Styrol und seinen Derivaten, Vinylchlorid, Vinylidenchlorid, Vinylacetat, Acrylderivaten gewählt sind, oder Copolymerisate dieser Monomeren untereinander und/oder mit modifizierend wirkenden Vinyl-Comonomeren sind, die bis zu 5 Gew.-% des Copolymerisats ausmachen.

3. Reagenz nach Anspruch 1 oder 2, dadurch gekennzeichnet, dass die Dienpolymerisate Homopolymerisate des Butadiens und seiner Derivate oder Copolymerisate dieser Monomeren untereinander und/oder mit Vinylmonomeren und/oder modifizierend wirkenden Vinylcomonomeren, die bis zu 5 Gew.-% des Copolymerisats ausmachen, sind.

4. Reagenz nach einem der Ansprüche 1 bis 3, dadurch gekennzeichnet, dass die Vinylpolymerisate, die die äussere Schicht bilden, Homopolymerisate des Styrols und seiner Derivate, von Alkylacrylaten und Alkylmethacrylaten (Alkyl mit 1 bis 10 Kohlenstoffatomen), von Acrylnitril oder Methacrylnitril, sowie Copolymerisate dieser Monomeren untereinander und/oder mit modifizierend wirkenden Vinylcomonomeren sind, die bis zu 5 Gew.-% des Copolymerisates ausmachen.

5. Reagenz nach einem der Ansprüche 1 bis 4, dadurch gekennzeichnet, dass das Kernpolymerisat 30 bis 99,5 Gew.-% der Partikel und das Polymerisat der äusseren Schicht 70 bis 0,5 Gew.-% der Partikel ausmacht.

6. Reagenz nach einem der Ansprüche 1 bis 5, dadurch gekennzeichnet, dass das immunologische aktive Material an einen gemäss Beispiel 1 hergestellten Latex gebunden ist.

7. Reagenz nach einem der Ansprüche 1 bis 6, dadurch gekennzeichnet, dass die Menge des immunologisch aktiven Materials 0,01 bis 15,0 Gew.-% beträgt.

8. Reagenz nach einem der Ansprüche 1 bis 7, dadurch gekennzeichnet, dass das immunologisch aktive Material kovalent an die diskreten Teilchen des Latexpolymeren gebunden ist.

9. Reagenz nach einem der Ansprüche 1 bis 8, dadurch gekennzeichnet, dass das immunologisch aktive Material humanes Immunoglobulin G ist.

10. Reagenz nach einem der Ansprüche 1 bis 8, dadurch gekennzeichnet, dass das immunologisch aktive Material Ziegen anti-Humanalbumin Immunoglobulin G ist.

11. Reagenz nach einem der Ansprüche 1 bis 8, dadurch gekennzeichnet, dass das immunologisch aktive Material Schaf anti-Human IgG Immunoglobulin G ist.

12. Verfahren zur Herstellung eines Reagens nach einem der Ansprüche 1 bis 11, dadurch gekennzeichnet, dass man den Latex nach Diazotierung oder unter Verwendung von geeigneten bifunktionellen Verbindungen mit dem immunologisch aktiven Material umsetzt.

13. Verfahren nach Anspruch 12, dadurch gekennzeichnet, dass man den gemäss Beispiel 1 hergestellten Latex nach Diazotierung mit denaturiertem humanem Immunoglobulin G umsetzt.

14. Verfahren nach Anspruch 12, dadurch gekennzeichnet, dass man den gemäss Beispiel 1 hergestellten Latex nach Diazotierung mit Ziegen anti-Humanalbumin Immunoglobulin G umsetzt.

15. Verfahren nach Anspruch 12, dadurch gekennzeichnet, dass man den gemäss Beispiel 1 hergestellten Latex unter Verwendung von p-Phenyldiisothiocyanat mit Schaf anti-Human IgG Immunoglobulin G umsetzt.

16. Verfahren nach Anspruch 12, dadurch gekennzeichnet, dass man den gemäss Beispiel 1 hergestellten Latex unter Verwendung von Bis-diazobenzidin mit humanem Immunoglobulin G umsetzt.

17. Verfahren zur Bestimmung einer immunologisch aktiven Substanz in einer Probe, dadurch gekennzeichnet, dass man die Probe und die mit dem entsprechenden immunologischen Reaktionspartner beschichteten Teilchen eines in den Ansprüchen 1 bis 5 definierten Latex vermischt und das Auftreten einer Agglutination beobachtet.

18. Verfahren zur Bestimmung einer immunologisch aktiven Substanz in einer Probe, dadurch gekennzeichnet, dass man die Probe mit einer bestimmten Menge des entsprechenden immunologischen Reaktionspartners und mit der immunologischen aktiven Substanz beschichteten Teilchen eines in den Ansprüchen 1 bis 5 definierten Latex vermischt und das Auftreten einer Agglutination beobachtet.

19. Verfahren nach Anspruch 17 zur Bestimmung des Rheumatoidfaktors in einer Probe, dadurch gekennzeichnet, dass man die Probe und mit denaturiertem humanen Immunoglobulin G beschichteten Teilchen eines gemäss Beispiel 1 hergestellten Latex vermischt und das Auftreten einer Agglutination beobachtet.

20. Verfahren nach Anspruch 17 zur Bestimmung von Humanalbumin in einer Probe, dadurch gekennzeichnet, dass man die Probe und mit Ziegen anti-Humanalbumin Immunoglobulin G beschichteten Teilchen eines gemäss Beispiel 1 hergestellten Latex vermischt und das Auftreten einer Agglutination beobachtet.

21. Verfahren nach Anspruch 17 zur Bestimmung von Human Immunoglobulin G in einer Probe, dadurch gekennzeichnet, dass man die Probe und mit Schaf anti-Human IgG-Immunoglobulin G beschichteten Teilchen eines gemäss Beispiel 1 hergestellten Latex vermischt und das Auftreten einer Agglutination beobachtet.

22. Verfahren nach Anspruch 18 zur Bestimmung von Human Immunoglobulin G in eine Probe, dadurch gekennzeichnet, dass man die Probe mit anti-Human-Immunoglobulin G Serum und mit Human Immunoglobulin G beschichteten Teilchen eines gemäss Beispiel 1 hergestellten Latex vermischt und das Auftreten einer Agglutination beobachtet.

23. Reagenziengarnitur zur Bestimmung einer immunologisch aktiven Substanz in einer Probe enthaltend in einem Behälter eine wässrige Suspension von mit dem entsprechenden immunologischen Reaktionspartner beschichteten Teilchen eines in den Ansprüchen 1 bis 5 definierten Latex.

24. Reagenziengarnitur zur Bestimmung einer immunologisch aktiven Substanz in einer Probe enthaltend in einem ersten Behälter eine Lösung des entsprechend immunologischen Reaktionspartners und in einem zweiten Behälter eine wässrige Suspension von mit dem immunologisch aktiven Material beschichteten Teilchen eines in den Ansprüchen 1 bis 5 definierten Latex.

25. Verwendung von einem in den Ansprüchen 1 bis 5 definierten Latex als Träger für immunologische Bestimmungen.

26. Verwendung eines Reagens nach einem der Ansprüche 1 bis 11 in immunologischen Bestimmungen.

***

Europäisches Patentamt

# EUROPÄISCHER RECHERCHENBERICHT

Nummer der Anmeldung
EP 78 10 0581

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A - 1 806 418 (MILES LABORATORIES)<br>* Seite 24, Ansprüche 1,2; Seite 6, Zeilen 10-16; Seite 5, Zeilen 1-4; Seite 25, Anspruch 7 *<br>-- | 1,16 |
| A | DE - A - 2 204 684 (HOFFMANN-LA ROCHE)<br>* Seite 4, Zeilen 29-34; Seite 5, Zeilen 1-4; Seite 21, Ansprüche 3,4 *<br>-- | 1,2,9,10 |
| A | US - A - 3 857 931 (H.J. HAGER)<br>* Spalte 10, Ansprüche 1,4 *<br>-- | 1,2,7,9 |
| A | FR - A - 2 331 567 (INSTIUT NATIONAL DE LA SOCIETE ET DE LA RECHERCHE MEDICALE-INSERM)<br>* Seite 14, Ansprüche 1,2 *<br>---- | 1,2 |

KLASSIFIKATION DER ANMELDUNG (Int.Cl.²)

G 01 N 33/16
C 08 F 8/00

RECHERCHIERTE SACHGEBIETE (Int. Cl.²)

G 01 N 33/16

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 13-11-1978 | ERTS |